# EUROPEAN PATENT APPLICATION

(11) **EP 4 442 805 A1**
(43) Date of publication of application: **09.10.2024**
(21) Application number: 22901707.4
(22) Date of filing: 28.11.2022
(51) Int. Cl.: C12N 1/20, C12P 17/18, A61K 31/4985, A61P 35/00, C12R 1/46

(54) **NOVEL ENTEROCOCCUS FAECIUM LCM001 STRAIN, AND USE THEREOF**

(30) Priority: 30.11.2021 KR 20210168581
(71) Applicant: Mbiometherapeutics Co., Ltd., Yongin-si, Gyeonggi-do 16950 (KR)
(72) Inventor: KANG, Se Chan, Suwon-si, Gyeonggi-do 16710 (KR); LEE, Yeong Geun, Suwon-si, Gyeonggi-do 16692 (KR); LEE, Hee Ju, Seongnam-si, Gyeonggi-do 13595 (KR)
(74) Representative: M. Zardi & Co S.A.
(86) International application number: PCT/KR2022/018994
(87) International publication number: WO 2023/101349

(57) **Abstract**

The present invention relates to a novel strain of Enterococcus faecium LCM001 and use thereof. Culture medium of the novel strain of Enterococcus faecium LCM001 (Deposited as KCTC 14669BP) may inhibit expression of any one of EGFR, ALK, KRAS, ROS1, HER2, RET, MET, BRAF, PIK3CA, NTRK1, FGFR, or DDR2 genes in cancer cells, and thereby inhibit human cancer cell viability and induce apoptosis. Therefore, the novel strain may be useful as a preventive and therapeutic agent for cancer.

## Description

### [FIELD OF THE INVENTION]

The present invention relates to a novel enterococcus faecium lcm001 strain, and use thereof.

### [BACKGROUND OF THE INVENTION]

Cancer is a disease that has not yet been conquered. Depending on the extent of metastasis and recurrence, treatment is impossible, and it is a disease causing high death rate worldwide. In Korea, the death rate from cancer has been steadily increasing every year. In 2012, there were an estimated 14.1 million new cases of cancer worldwide, with 8.5 million deaths from cancer (Ferlay et al., 2013). According to the 2019 Statistics Korea 'Causes of Death Statistics', cancer is reported as the most threatening disease, accounting for the highest death rate. It can occur in various parts of the body, including several organs and bones or lymph nodes, and can spread to surrounding tissues and other organs, causing serious symptoms (WHO, 2006).

Currently, surgery, radiation therapy and chemotherapy are used for the treatment of cancer. Among these, chemotherapy refers to a method of treating cancer using anticancer drugs. Today, there are around 60 different anticancer drugs being used, and research on the development of new anticancer drugs is actively progressing as more knowledge about cancer occurrence and characteristics of cancer cells is informed.

Many cancer patients suffer from pain due to side effects of chemotherapy, and limited administration is being carried out due to the toxicity of chemotherapy drugs. Anticancer agents used in clinical practice not only affect cancer cells but also normal cells, leading to problems such as side effects and drug resistance as the number of administrations increases, resulting in treatment failure. Research is in progress for developing new forms of anticancer agents that can overcome the side effects of existing anticancer agents caused by the diversity of cancer itself and the diversification of the mechanisms.

Although the current viability rates for cancer are significantly higher than in the past, cancer remains one of the diseases that is difficult to completely conquer. In particular, when cancer occurs, treatment typically involves surgical procedures, radiation therapy and chemotherapy. However, even with these treatments, completely removing microscopic lesions that have invaded surrounding tissues or metastasized to lymph nodes are difficult.

### [DETAILED DESCRIPTION OF THE INVENTION]

### [PROBLEMS TO BE SOLVED]

The present invention aims to provide a novel strain of Enterococcus faecium LCM001 (Deposition number: KCTC 14669BP). Additionally, it aims to provide a composition for cancer treatment containing Enterococcus faecium LCM001 (Deposition number: KCTC 14669BP), a culture medium, or a non-cellular supernatant as an active ingredient. Furthermore, the present invention aims to provide a composition for cancer treatment containing Cyclo(L-Pro-L-Tyr) and its derivatives, isolated from Enterococcus faecium LCM001 (Deposition number: KCTC 14669BP) culture medium or non-cellular supernatant, as an active ingredient.

### [SOLUTION]

To achieve the objectives, the present invention provides a novel strain of Enterococcus faecium LCM001 (Deposition number: KCTC 14669BP). The strain is characterized by having a 16S rRNA gene sequence [SEQ ID NO: 1].

[SEQ ID NO: 1]

GTTGGATCTTGGATCAGGACGAACGCTGGCGGCGTGCCTAATACATGCAAG

TCGTACGCTTCTTTTTCCACCGGAGCTTGCTCCACCGGAAAAAGAAGAGTGG

CGAACGGGTGAGTAACACGTGGGTAACCTGCCCATCAGAAGGGGATAACAC

TTGGAAACAGGTGCTAATACCGTATAACAATCGAAACCGCATGGTTTTGATT

TGAAAGGCGCTTTCGGGTGTCGCTGATGGATGGACCCGCGGTGCATTAGCT

AGTTGGTGAGGTAACGGCTCACCAAGGCCACGATGCATAGCCGACCTGAGA
GGGTGATCGGCCACATTGGGACTGAGACACGGCCCAAACTCCTACGGGAGG
CAGCAGTAGGGAATCTTCGGCAATGGACGAAAGTCTGACCGAGCAACGCCG
CGTGAGTGAAGAAGGTTTTCGGATCGTAAAACTCTGTTGTTAGAGAAGAAC
AAGGATGAGAGTAACTGTTCATCCCTTGACGGTATCTAACCAGAAAGCCAC
GGCTAACTACGTGCCAGCAGCCGCGGTAATACGTAGGTGGCAAGCGTTGTC
CGGATTTATTGGGCGTAAAGCGAGCGCAGGCGGTTTCTTAAGTCTGATGTG
AAAGCCCCCGGCTCAACCGGGGAGGGTCATTGGAAACTGGGAGACTTGAGT
GCAGAAGAGGAGAGTGGAATTCCATGTGTAGCGGTGAAATGCGTAGATATA
TGGAGGAACACCAGTGGCGAAGGCGGCTCTCTGGTCTGTAACTGACGCTGA
GGCTCGAAAGCGTGGGGAGCAAACAGGATTAGATACCCTGGTAGTCCACGC
CGTAAACGATGAGTGCTAAGTGTTGGAGGGTTTCCGCCCTTCAGTGCTGCAG
CTAACGCATTAAGCACTCCGCCTGGGGAGTACGACCGCAAGGTTGAAACTC
AAAGGAATTGACGGGGGCCCGCACAAGCGGAGGAGCCTGTGGTTTAATTCG
AACCAACGCGAACAACCTTACCAGGTCTTGACATCCTTTGACCACTCCAGA
GATAGACCTTCCCCTTCGGGGGCAAAGTGACAGGTGGTGCATGGTTGTCGT
CAGCTCGTGTCGTGAGATGTTGGGTTAAGTCCCGCAACGAGCGCAACCCTT
ATTGTTAGTTGCCATCATTCAGTTGGGCACTCTAGCAAGACTGCCGGTGACA
AACCGGAGGAAGGTGGGGATGACGTCAAATCATCATGCCCCTTATGACCTG
GGCTACACACGTGCTACAATGGGAAGTACAACGAGTTGCGAAGTCGCGAGG
CTAAGCTAATCTCTTAAAGCTTCTCTCAGTTCGGATTGCAGGCTGCAACTCG
CCTGCATGAAGCCGGAATCGCTAGTAATCGCGGATCAGCACGCCGCGGTGA
ATACGTTCCCGGGCCTTGTACACACCGCCCGTCACACCACGAGAGTTTGTAA
CACCAGATGTCGGTGAGGTAACC.

The culture medium or cell-free supernatant of Enterococcus faecium LCM001 strain shows apoptosis of cancer cells. The culture medium or cell-free supernatant of Enterococcus faecium LCM001 strain may inhibit the expression of any one of EGFR, ALK, KRAS, ROS1, HER2, RET, MET, BRAF, PIK3CA, NTRK1, FGFR, or DDR2 in cancer cells, but is not limited thereto.

The culture medium or cell-free supernatant of Enterococcus faecium LCM001 strain specifically induces apoptosis of cancer cells without affecting normal cells.

The culture medium or cell-free supernatant of Enterococcus faecium LCM001 strain may inhibit EGFR/KRAS/PI3K pathway in cancer cells, but is not limited thereto.

According to another embodiment of the present invention, the present invention provides a composition for cancer prevention or treatment, comprising a novel strain of Enterococcus faecium LCM001 (Deposition number: KCTC 14669BP), culture medium, or cell-free supernatant of the novel strain as an active ingredient.

The culture medium or cell-free supernatant of the strain may inhibit the expression of any one of EGFR, ALK, KRAS, ROS1, HER2, RET, MET, BRAF, PIK3CA, NTRK1, FGFR, or DDR2 in cancer cells, but is not limited thereto.

According to another embodiment of the present invention, the present invention provides a composition for cancer prevention or treatment, comprising a compound represented by the following [Chemical Formula 1] as an active ingredient: 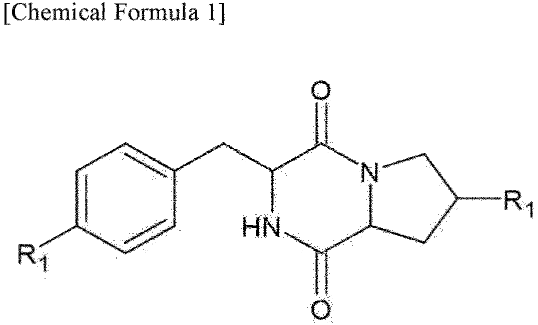 R1 is H or OH, independently.

The compound [Chemical Formula 1] may be isolated from the culture medium of the novel strain of Enterococcus faecium LCM001 (Deposition number: KCTC 14669BP), but is not limited thereto.

The compound [Chemical Formula 1] may inhibit the expression of any one of EGFR, ALK, KRAS, ROS1, HER2, RET, MET, BRAF, PIK3CA, NTRK1, FGFR, or DDR2 in cancer cells, but is not limited thereto.

The compound may be any one of [Chemical Formula 2] to [Chemical Formula 6], but is not limited thereto.

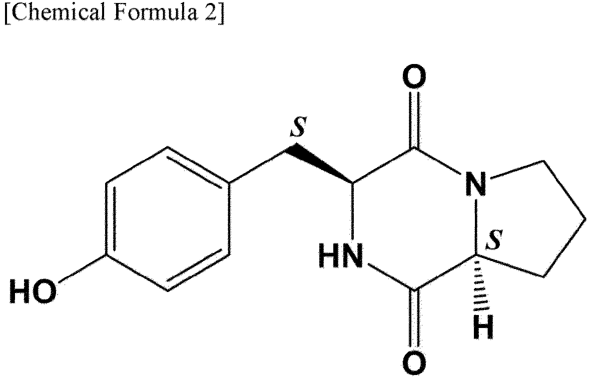

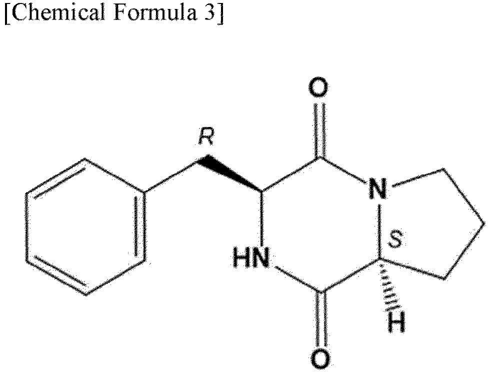

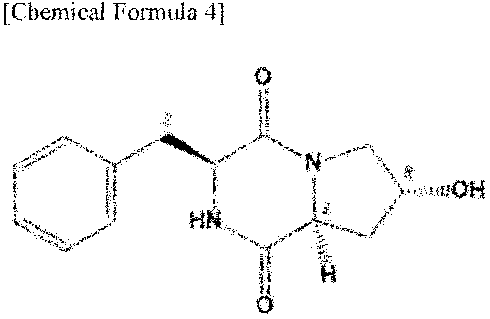

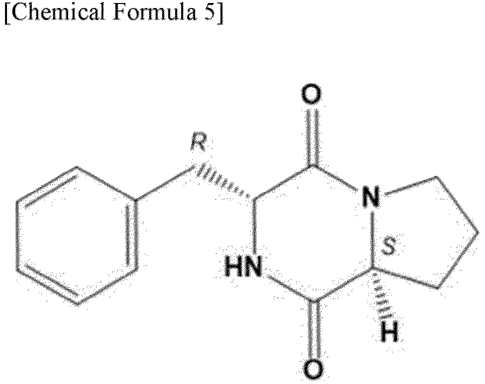

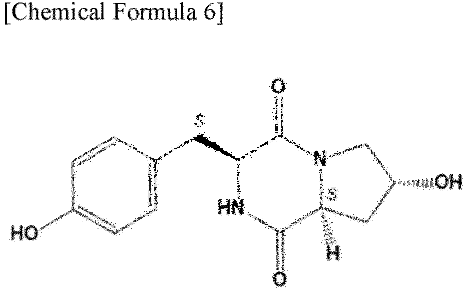

According to another embodiment of the present invention, the present invention provides a composition for cancer prevention or improvement, food composition, or health functional food, comprising a novel strain of Enterococcus faecium LCM001 (Deposition number: KCTC 14669BP), culture medium, or cell-free supernatant of the strain as an active ingredient.

Another embodiment of the present invention, the present invention provides a composition for cancer prevention or improvement, food composition, or health functional food, comprising a compound represented by the following [Chemical Formula 1] as an active ingredient: 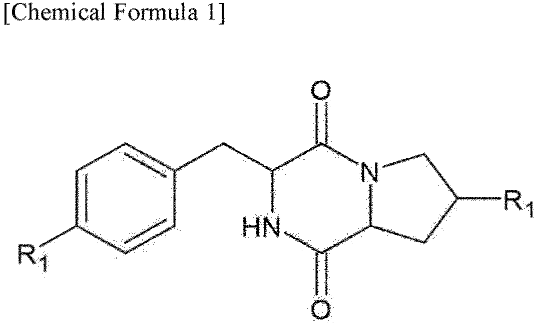 R1 is H or OH, independently.

The cancer may be one or more selected from the group consisting of cervical cancer, lung cancer, pancreatic cancer, non-small cell lung cancer, liver cancer, colon cancer, colorectal cancer, bone cancer, skin cancer, brain cancer, throat cancer, skin melanoma, intraocular melanoma, uterine cancer, ovarian cancer, rectal cancer, liver cancer, brain tumor, bladder cancer, blood cancer, stomach cancer, anal cancer, breast cancer, fallopian tube cancer, endometrial cancer, vaginal cancer, vulvar cancer, Hodgkin's disease, esophageal cancer, small intestine cancer, endocrine gland cancer, thyroid cancer, parathyroid cancer, adrenal gland cancer, sarcoma, urethral cancer, penile cancer, prostate cancer, bladder cancer, kidney cancer, ureter cancer, renal cell carcinoma, renal pelvis carcinoma, central nervous system (CNS) tumors, primary CNS lymphoma, spinal cord tumors, brainstem gliomas, and pituitary gland tumors, but is not limited thereto.

### [BENEFITS OF THE INVENTION]

The culture medium of a novel strain of Enterococcus faecium LCM001 (deposition number: KCTC 14669BP) according to the present invention may inhibit the expression of any one of EGFR, ALK, KRAS, ROS1, HER2, RET, MET, BRAF, PIK3 CA, NTRK 1, FGFR, or DDR2 in cancer cells, and thereby suppress the viability of human cancer cells and induce apoptosis. As a result, the novel strain of Enterococcus faecium LCM001 (deposition number: KCTC 14669BP) culture medium may be effectively utilized as a cancer prevention and treatment agent.

### [BRIEF DESCRIPTION OF DRAWINGS]

FIG.1 depicts genetic phylogenetic analysis of the novel strain of Enterococcus faecium LCM001 (Deposition number: KCTC 14669BP). The strain belongs to Enterococcus genus and shows 85% similarity to Enterococcus faecium.
FIG. 2A depicts cell-free supernatant of the novel strain of Enterococcus faecium LCM001 was used to show the cell viability rates of lung cancer cells (A549) and normal lung cells (Wi38) in type of a graph. The novel strain inhibited the growth of lung cancer cells (A549) only and induced apoptosis.
FIG. 2B depicts cell-free supernatant of the novel strain of Enterococcus faecium LCM001 was used to show the cell viability rates of colon cancer cells (HCT-15), liver cancer cells (Sk-hep-1), pancreatic cancer cells (PANC-1), and breast cancer cells (MCF-7) in type of a graph. Similar to lung cancer cells (A549), the strain inhibited the growth of other cancer cells and induced apoptosis.
FIG. 3 depicts a mechanism causing to cell proliferation suppression and induction of apoptosis according to EGFR/KRAS inhibition.

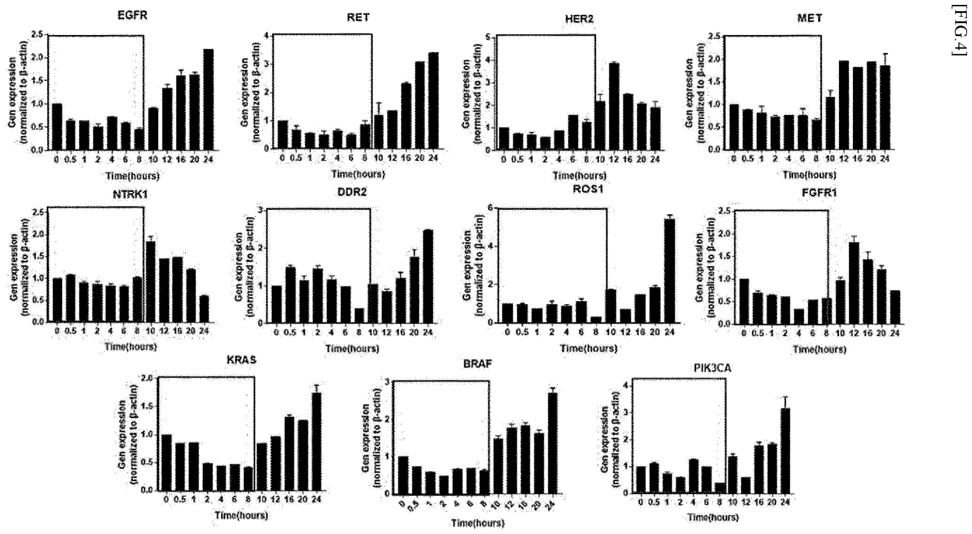

FIG. 4 depicts a graph of fold change in mRNA expression level normalized to β-actin, targeting the genes listed in Table 1, which are key biomarkers targeting therapeutically to human non-small cell lung cancer. The fold change is compared to the control group.
FIG. 5 depicts a graph of the result of Hollow Fiber Assay (HFA) using the cell-free supernatant of the novel strain of Enterococcus faecium LCM001, showing proliferation inhibition in lung cancer cells.
FIG. 6 depicts a schematic diagram illustrating a process of isolating the active ingredient (Cyclo(L-Pro-L-Tyr)) from the cell-free supernatant of the novel strain of Enterococcus faecium LCM001.
FIG. 7 depicts the compound Cyclo(L-Pro-L-Tyr) isolated from the cell-free supernatant of the novel strain of Enterococcus faecium LCM001.
FIG. 8 depicts a graph of IC50 values obtained from MTT assay using derivatives of the active ingredient Cyclo(L-Pro-L-Tyr).

### [A MODE FOR IMPLEMENTING THE INVENTION]

The structural or functional descriptions provided for the embodiments disclosed in the present specification or application are merely illustrative for the purpose of describing examples according to the present invention, and the embodiments according to the present invention may be implemented in various forms, and should not be interpreted as limited to the embodiments described in the present specification or application.

Embodiments according to the present invention may be subject to various modifications and may take various forms, so specific embodiments are illustrated in the drawings and described in detail in the present specification or application. However, this is not intended to limit the embodiments according to the concept of the present invention to specific embodiments, and should be understood to include all modifications, equivalents, and alternatives falling within the scope of the present invention.

Unless otherwise defined, all technical or scientific terms used herein have the same meaning as commonly understood by one of ordinary skill in the art to which the present invention belongs. Terms commonly used in the art are to be interpreted as having the meanings consistent with the meanings they have in the context of the relevant art, and are not to be interpreted in an idealized or overly formal sense unless clearly defined in the present specification.

The present invention provides a novel strain of Enterococcus faecium LCM001. The inventors identified a microorganism with excellent anti-cancer activity and determined it to be a novel strain of Enterococcus faecium LCM001. Culture medium or cell-free supernatant of the novel strain of Enterococcus faecium LCM001 exhibits excellent cell apoptotic effect on cancer cells.

The inventors analyzed 16s rRNA sequence of the novel strain of Enterococcus faecium LCM001 and, through genetic phylogenetic analysis, confirmed that the novel strain belongs to a genus of Enterococcus and shows 85% similarity to Enterococcus faecium. The novel strain of Enterococcus faecium LCM001 was deposited with Korean Collection for Type Cultures (KCTC) on August 23, 2021, under deposition number KCTC 14669BP.

The 16S rRNA sequence of the novel strain of Enterococcus faecium LCM001 is SEQ ID NO: 1.

[SEQ ID NO: 1]
GTTGGATCTTGGATCAGGACGAACGCTGGCGGCGTGCCTAATACATGCAAG
TCGTACGCTTCTTTTTCCACCGGAGCTTGCTCCACCGGAAAAAGAAGAGTGG
CGAACGGGTGAGTAACACGTGGGTAACCTGCCCATCAGAAGGGGATAACAC
TTGGAAACAGGTGCTAATACCGTATAACAATCGAAACCGCATGGTTTTGATT
TGAAAGGCGCTTTCGGGTGTCGCTGATGGATGGACCCGCGGTGCATTAGCT
AGTTGGTGAGGTAACGGCTCACCAAGGCCACGATGCATAGCCGACCTGAGA
GGGTGATCGGCCACATTGGGACTGAGACACGGCCCAAACTCCTACGGGAGG
CAGCAGTAGGGAATCTTCGGCAATGGACGAAAGTCTGACCGAGCAACGCCG
CGTGAGTGAAGAAGGTTTTCGGATCGTAAAACTCTGTTGTTAGAGAAGAAC
AAGGATGAGAGTAACTGTTCATCCCTTGACGGTATCTAACCAGAAAGCCAC
GGCTAACTACGTGCCAGCAGCCGCGGTAATACGTAGGTGGCAAGCGTTGTC
CGGATTTATTGGGCGTAAAGCGAGCGCAGGCGGTTTCTTAAGTCTGATGTG
AAAGCCCCCGGCTCAACCGGGGAGGGTCATTGGAAACTGGGAGACTTGAGT
GCAGAAGAGGAGAGTGGAATTCCATGTGTAGCGGTGAAATGCGTAGATATA
TGGAGGAACACCAGTGGCGAAGGCGGCTCTCTGGTCTGTAACTGACGCTGA
GGCTCGAAAGCGTGGGGAGCAAACAGGATTAGATACCCTGGTAGTCCACGC
CGTAAACGATGAGTGCTAAGTGTTGGAGGGTTTCCGCCCTTCAGTGCTGCAG
CTAACGCATTAAGCACTCCGCCTGGGGAGTACGACCGCAAGGTTGAAACTC
AAAGGAATTGACGGGGGCCCGCACAAGCGGAGGAGCCTGTGGTTTAATTCG
AACCAACGCGAACAACCTTACCAGGTCTTGACATCCTTTGACCACTCCAGA
GATAGACCTTCCCCTTCGGGGGCAAAGTGACAGGTGGTGCATGGTTGTCGT
CAGCTCGTGTCGTGAGATGTTGGGTTAAGTCCCGCAACGAGCGCAACCCTT
ATTGTTAGTTGCCATCATTCAGTTGGGCACTCTAGCAAGACTGCCGGTGACA
AACCGGAGGAAGGTGGGGATGACGTCAAATCATCATGCCCCTTATGACCTG
GGCTACACACGTGCTACAATGGGAAGTACAACGAGTTGCGAAGTCGCGAGG
CTAAGCTAATCTCTTAAAGCTTCTCTCAGTTCGGATTGCAGGCTGCAACTCG
CCTGCATGAAGCCGGAATCGCTAGTAATCGCGGATCAGCACGCCGCGGTGA

ATACGTTCCCGGGCCTTGTACACACCGCCCGTCACACCACGAGAGTTTGTAA
CACCAGATGTCGGTGAGGTAACC.

The culture medium or cell-free supernatant of the novel of Enterococcus faecium LCM001 inhibits the growth of cancer cells and exhibits a cell apoptosis effect. According to the experiments conducted by the inventors, the culture medium or cell-free supernatant of the novel strain of Enterococcus faecium LCM001 inhibits the expression of any one of EGFR (epidermal growth factor receptor), ALK, KRAS (Kirsten rat sarcoma viral oncogene homolog), ROS1 (ROS Proto-Oncogene 1, Receptor Tyrosine Kinase), HER2 (Erb-b2 receptor tyrosine kinase 2), RET (Ret Proto-Oncogene), MET (MET Proto-Oncogene, Receptor Tyrosine Kinase), BRAF (serine/threonine-protein kinase B-Raf), PIK3CA (phosphatidylinositol-4,5-bisphosphate 3-kinase catalytic subunit alpha), NTRK1 (Neurotrophic Receptor Tyrosine Kinase 1), FGFR (Fibroblast Growth Factor Receptor 1), or DDR2 (Discoidin Domain Receptor Tyrosine Kinase) in cancer cells. Consequently, the novel strain activates cell death by inhibiting the EGFR/KRAS/PI3K pathway in cancer cells.

The cancer may be one or more selected from the group consisting of cervical cancer, lung cancer, pancreatic cancer, non-small cell lung cancer, liver cancer, colorectal cancer, colon cancer, bone cancer, skin cancer, brain cancer, throat cancer, skin melanoma, intraocular melanoma, uterine cancer, ovarian cancer, rectal cancer, liver cancer, brain tumors, bladder cancer, blood cancer, stomach cancer, anal area cancer, breast cancer, fallopian tube cancer, endometrial cancer, vaginal cancer, vulvar cancer, Hodgkin's disease, esophageal cancer, small intestine cancer, endocrine gland cancer, thyroid cancer, parathyroid cancer, adrenal gland cancer, connective tissue sarcoma, ureter cancer, penile cancer, prostate cancer, bladder cancer, kidney cancer, ureter cancer, renal cell carcinoma, renal pelvis carcinoma, central nervous system (CNS) tumors, primary CNS lymphoma, spinal cord tumors, brainstem glioma, and pineal gland tumors, but is not limited thereto.

The culture medium or cell-free supernatant of the novel of Enterococcus faecium LCM001 exhibited apoptotic effect on various cancer cells but not on normal lung cells. Therefore, according to another embodiment of the present invention, the present invention provides a composition for preventing or treating cancer, comprising a novel strain of Enterococcus faecium LCM001 (Deposited as KCTC 14669BP), culture medium or cell-free supernatant thereof as an active ingredient. The inventors have isolated an active ingredient showing apoptosis effects on cancer cells from the culture medium or cell-free supernatant of the novel strain, and the compound can be represented by the following [Chemical formula 1]. 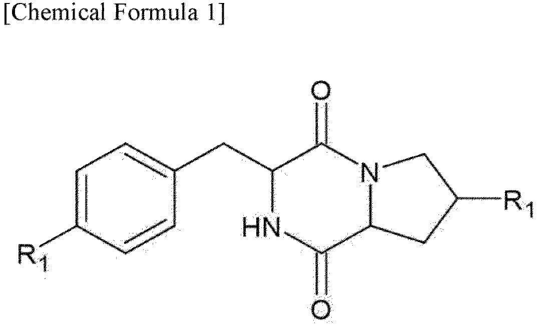 R1 is H or OH, independently.

Furthermore, the inventors have confirmed that the apoptotic effect on cancer cells is higher when the number of -OH groups is lower. Therefore, according to another embodiment of the present invention, the present invention provides a composition for preventing or treating cancer, comprising a compound represented by [Chemical formula 1] as an active ingredient.

Specifically, the inventors have isolated Cyclo(L-Pro-L-Tyr) as an active ingredient showing apoptosis effects on cancer cells from the culture medium or cell-free supernatant of the novel strain of Enterococcus faecium LCM001. The Cyclo(L-Pro-L-Tyr) has a chemical structure represented by [Chemical formula 2].

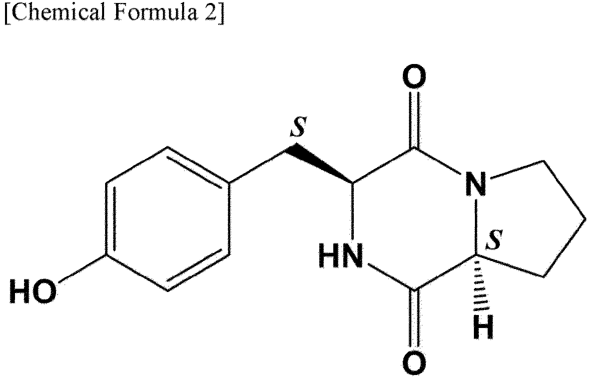

Furthermore, the inventors have confirmed that derivatives of Cyclo(L-Pro-L-Tyr) represented by [Chemical formula 3] to [Chemical formula 6] exhibit apoptotic effects on cancer cells.

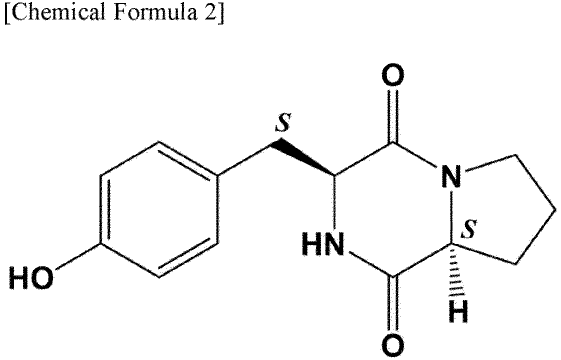

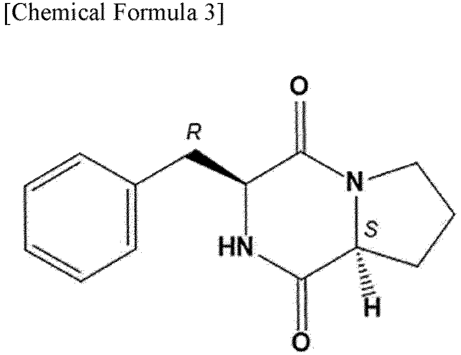

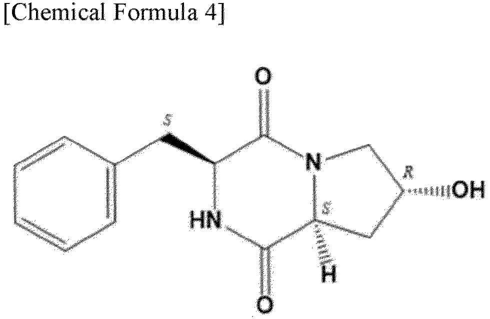

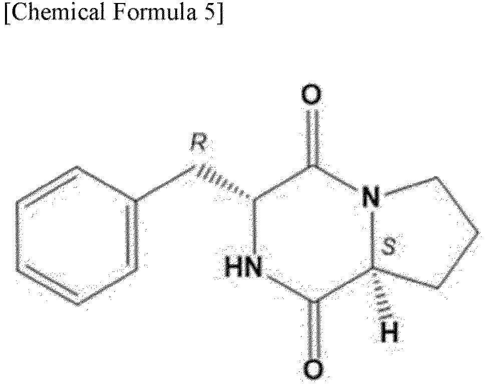

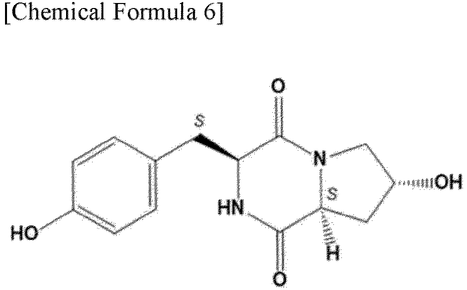

Therefore, according to another embodiment of the present invention, the present invention provides a composition for cancer prevention or treatment comprising a compound represented by [Chemical Formula 2] to [Chemical Formula 6] as an active ingredient.

The cancer may be one or more selected from the group consisting of cervical cancer, lung cancer, pancreatic cancer, non-small cell lung cancer, liver cancer, colorectal cancer, colon cancer, bone cancer, skin cancer, brain cancer, throat cancer, skin melanoma, intraocular melanoma, uterine cancer, ovarian cancer, rectal cancer, liver cancer, brain tumors, bladder cancer, blood cancer, stomach cancer, anal area cancer, breast cancer, fallopian tube cancer, endometrial cancer, vaginal cancer, vulvar cancer, Hodgkin's disease, esophageal cancer, small intestine cancer, endocrine gland cancer, thyroid cancer, parathyroid cancer, adrenal gland cancer, connective tissue sarcoma, ureter cancer, penile cancer, prostate cancer, bladder cancer, kidney cancer, ureter cancer, renal cell carcinoma, renal pelvis carcinoma, central nervous system (CNS) tumors, primary CNS lymphoma, spinal cord tumors, brainstem glioma, and pineal gland tumors, but is not limited thereto.

The composition for cancer prevention or treatment may be formulated conventionally into a form by selecting one or more pharmaceutically acceptable carriers or additives and an effective amount of the compound represented by [Chemical Formula 1], in addition to the main component, the novel strain of Enterococcus faecium LCM001, culture medium, or cell-free supernatant of the novel strain.

The carrier may be one or two or more selected from diluent, solvent, binder, disintegrant, sweetener, stabilizer and preservative, and the additive may be one or two or more selected from flavors, vitamins, and antioxidants. In the present invention, all pharmaceutically acceptable carriers and additives are usable. Specifically the diluent may be lactose monohydrate, trehalose, cornstarch, soybean oil, microcrystalline cellulose, or D-mannitol, and the lubricant may be magnesium stearate or talc is preferable, and the binder may be polyvinyl pyrrolidone (PVP) or hydroxypropylcellulose (HPC), and, the disintegrant carboxymethylcellulose calcium (Ca-CMC), sodium starch glycolate, polacrylin potassium, or cross-linked polyvinylpyrrolidone, and the sweetener may be dextrose, fructose, sorbitol, or aspartame is preferable as a sweetener, and the stabilizer may be carboxymethylcellulose sodium (Na-CMC), β-cyclodextrin, white bee's wax, or xanthan gum is preferable, and the preservative may be methyl p-hydroxy benzoate (methylparaben), propyl p-hydroxybenzoate (propylparaben), or potassium sorbate is preferable as a preservative, but this is not limited thereto.

The pharmaceutical composition of the invention may be administered to a patient in a single dose or may be administered in multiple doses over an extended period of time by a fractionated treatment protocol. The term "pharmaceutically effective amount" refers to an amount that produces a response greater than that produced by a negative control group and preferably an amount sufficient to prevent or treat inflammatory diseases. Additionally, the pharmaceutically effective amount can vary appropriately depending on various factors such as the severity of the disease, the patient's age, weight, health status, gender, route of administration, and duration of treatment.

The composition of the present invention may be formulated in various forms depending on the route of administration, using pharmaceutically acceptable carriers as well as methods known in the art. The term "pharmaceutically acceptable" refers to a composition that is physiologically tolerable, does not interfere with the action of the active ingredient when administered to humans, and does not cause toxicity such as gastrointestinal disorders, dizziness, allergic reactions, or similar reactions. The composition of the present invention may be formulated in various forms depending on the route of administration, using pharmaceutically acceptable carriers as well as methods known in the art. The composition can be administered orally or non-orally, but not limited thereto.

According to another embodiment of the present invention, the present invention provides a food composition for cancer prevention or improvement, comprising a novel strain of Enterococcus faecium LCM001 (Deposit number: KCTC 14669BP), culture medium, or cell-free supernatant of the novel strain as an active ingredient.

According to another embodiment of the present invention, the present invention provides a health functional food for cancer prevention or improvement, comprising a novel strain of Enterococcus faecium LCM001 (Deposit number: KCTC 14669BP), culture medium, or cell-free supernatant of the novel strain as an active ingredient.

According to another embodiment of the present invention, the present invention provides a food composition or health functional food for cancer prevention or improvement, comprising a compound represented by [Chemical Formula 1] as an active ingredient. 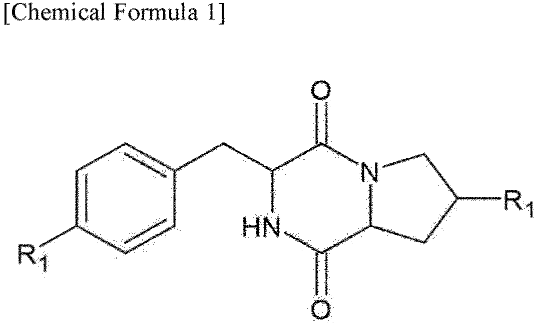 R1 is H or OH, independently.

The health functional food of the present invention may include beverages (including tea or alcoholic beverages), fruits and their processed foods (e.g., canned fruits, preserves, jams, marmalades, etc.), fish, meat and their processed foods (e.g., ham, sausage, corned beef, etc.), bread and noodles (e.g., udon, soba noodles, ramen, spaghetti, macaroni, etc.), juices, various drinks, cookies, taffy, dairy products (e.g., butter, cheese, etc.), edible plant oils, margarine, plant-based proteins, retort foods, frozen foods, various seasonings (e.g., miso, soy sauce, sauces, etc.), and the like.

Furthermore, the health functional food of the present invention may be formulated into various forms such as purified, refined, acidified, granulated, powdered, encapsulated, liquid, etc. The health functional food may be formulated with one or more of excipients, diluents, disintegrants, and additives.

Hereinafter, the composition and effects of the present invention are further described in detail based on examples. The examples are provided solely for the purpose of describing the present invention, and the scope of the present invention is not limited by the examples.

### Example 1. Apoptotic effect of a novel strain of Enterococcus faecium LCM001 on cancer cells

### <Culture of microorganism>

The microorganism, Enterococcus faecium LCM001 was cultured in an incubator at 37°C, 150 rpm for 24 hours after inoculating a single colony into Tryptic Soy Broth (TSB), being sterilized at 121°C for 15 minutes under high-pressure steam.

### <Microbial identification>

To identify the microorganism that inhibited the viability rate of cancer cells, Tryptic Soy Agar (TSA) culture medium were prepared in a petri dish, and the microorganism was smeared on the petri dish and cultured at 37°C for 24 hours. The microorganism was analyzed for 16S rRNA gene sequence thereof, which is commonly used to analyze the evolutionary relationships between species, by submitting the microorganism to the Korean Collection for Type Cultures (KCTC) for the analysis.

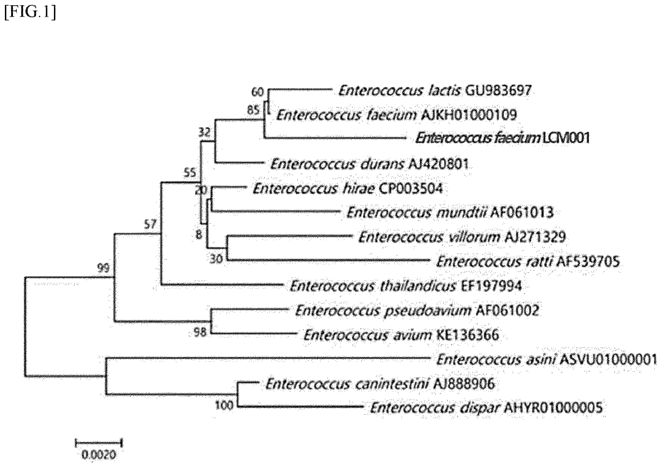

The inventors aimed to select a strain with apoptosis effects on cancer cells. Various strains isolated from human feces were tested to confirm apoptotic effect on cancer cells, and thereby a strain with superior apoptotic effects was selected. The 16S rRNA gene sequence of the selected strain was analyzed, revealing that the strain belonged to the Enterococcus genus and showed 85% homology with Enterococcus faecium(Fig. 1).

The selected strain was named Enterococcus faecium LCM001 and deposited at the Korean Collection for Type Cultures (KCTC) on August 23, 2021, under deposit number KCTC 14669BP.

### <Cell Culture>

The human non-small cell lung cancer (NSCLC) A549 cells were cultured in RPMI 1640 culture medium supplemented with 10% fetal bovine serum (FBS) and 1% penicillin/streptomycin in a incubator at 37°C in a 5% CO₂. Human normal fibroblast lung cell Wi38 were cultured in MEM culture medium supplemented with 10% FBS and 1% penicillin/streptomycin.

HCT-15 colon cancer cells were cultured in RPMI culture medium supplemented with 10% FBS and 1% penicillin/streptomycin. Sk-hep-1 liver cancer cells and PANC-1 pancreatic cancer cells were cultured in DMEM culture medium supplemented with 10% FBS and 1% penicillin/streptomycin. MCF-7 breast cancer cells were cultured in EMEM culture medium supplemented with 10% FBS and 1% penicillin/streptomycin.

### <Cell Viability>

The cell viability rate was measured using MTT [3-(4,5-dimethylthiazol-2-yl)-2,5-diphenyl-tetrazolium bromide] assay. Specifically, lung cancer cells (A549, 7×10³ cells/well), colon cancer cells (HCT-15, 5×10³ cells/well), liver cancer cells (Sk-hep-1, 5×10³ cells/well), pancreatic cancer cells (PANC-1, 5×10³ cells/well), and breast cancer cells (MCF-7, 2×10³ cells/well) were seeded in a 96-well culture plate at 3 times repeatedly, and cultured overnight at 37°C in 5% CO₂ with RPMI 1640 medium containing 10% FBS. Subsequently, the cell-free supernatant was added to each well at concentrations of 1X, 2X, 5X, and 10X, with 100 µL per well, and cultured for 24 hours at 37°C in 5% CO₂.

To assess cell apoptosis, MTT reagent at a concentration of 5 mg/ml was added to each well with 10 µL per well and reacted for 4 hours at 37°C in 5% CO₂. The cells were then dissolved in dimethyl sulfoxide (DMSO, 100 µL/well), and the absorbance at 570 nm was measured using a microplate reader. The cell viability rate was calculated as a percentage using the following formula. The cell toxicity test was conducted in triplicate, and the values are presented as mean ± standard deviation. Cell viability(%) = (the absorption of the treated group / the absorption of the control group) X 100

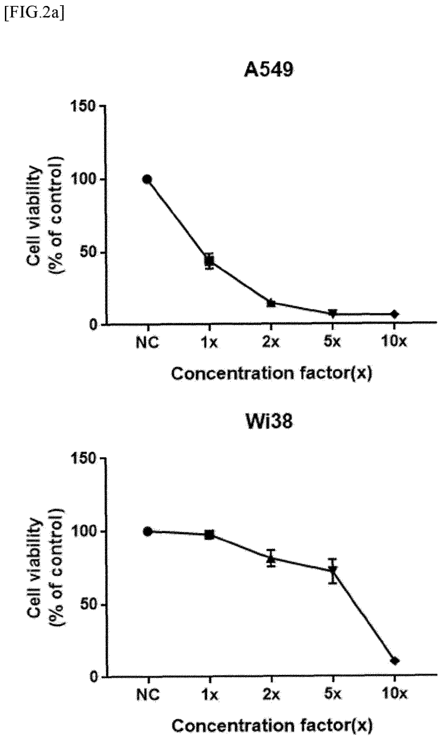

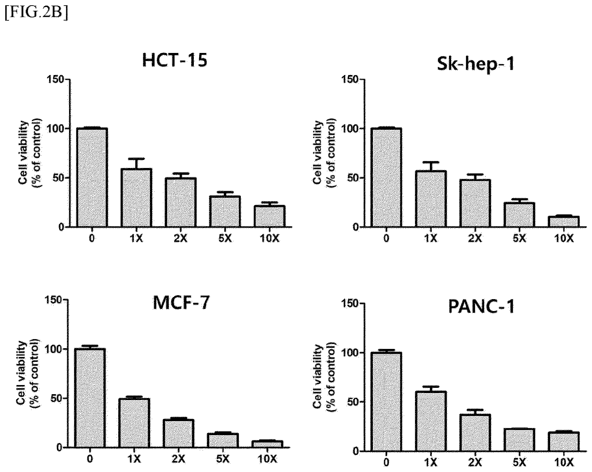

In FIG. 2, the cell-free supernatant of Enterococcus faecium LCM001 showed a concentration-dependent effect in inducing cell apoptosis in human non-small cell lung cancer cells (A549). However, any significant cell apoptotic effect was not shown on human normal lung fibroblast cells (Wi38) except at the 10X concentration.

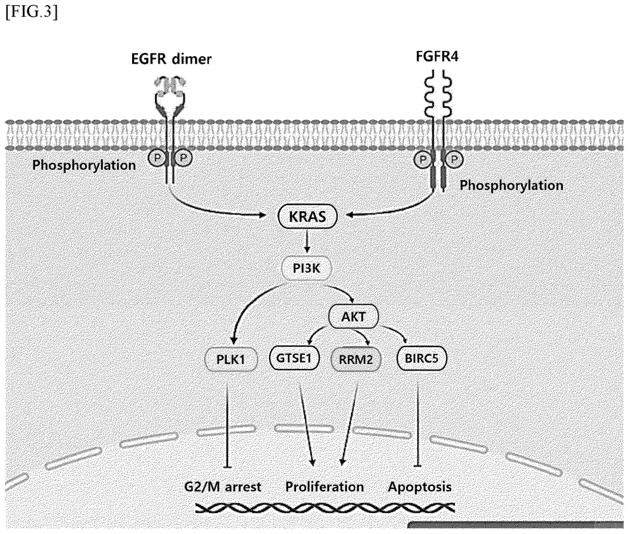

In FIG. 3, the cell-free supernatant of Enterococcus faecium LCM001 showed a similar cell apoptotic effect on colorectal cancer cells (HCT-15), liver cancer cells (Sk-hep-1), pancreatic cancer cells (PANC-1), and breast cancer cells (MCF-7) as observed in the non-small cell lung cancer cells (A549).

### <Extraction of total RNA>

To extract total RNA, lung cancer cells (A549, 1.2 × 10⁶ cells/well) were seeded in 6-well culture plates twice repeatedly and cultured overnight in RPMI 1640 medium supplemented with 10% FBS at 37°C with 5% CO₂. The culture medium was then replaced with FBS-free RPMI 1640 medium containing various sample concentrations, and RNase treatment was performed at 0, 2, 4, 6, 8, 10, 12, 16, 18, 20, and 24 hours.

After RNase treatment, 250 µL of chloroform was added and mixed vigorously. The mixture was then centrifuged at 12,000 rpm at 4°C for 15 minutes. After centrifugation, the supernatant was transferred to a new tube, and an equal volume of isopropanol was added. The mixture was slowly mixed and allowed to react for about 10 minutes, followed by centrifugation at 12,000 rpm at 4°C for 10 minutes.

After centrifugation, the supernatant was removed, and the precipitate was washed with 75% ethanol. The mixture was then centrifuged at 7,500 rpm for 5 minutes at 4°C. After centrifugation, the supernatant was removed, and the precipitate was dried. Once the 75% ethanol evaporated, the precipitate was dissolved in DEPC solution. The final purified total RNA was quantified using NanoQuant Plate^{™} by measuring absorbance at 260 nm and 280 nm and was used for RT-PCR.

### <RT-PCR>

The extracted total RNA (2 µg) was used as a template for cDNA synthesis using the PrimeScript^{™} 1st strand cDNA synthesis kit (TaKaRa, Japan) according to the manufacturer's manual. The synthesized cDNA was then used for qRT-PCR.

### <qRT-PCR analysis>

The human non-small cell lung cancer-specific genes that are overexpressed and the mutant genes that cause translocation were selected as major predictive biomarkers for the present invention, and major biomarkers targeting human non-small cell lung cancer as therapeutic targets are listed in [Table 1].

**[Table 1]**

| Gene | Alteration | Adenocarcinoma frequency(10%) |
|---|---|---|
| EGFR | Mutation | 10 |
| KRAS | Mutation | 25-35 |
| ROS1 | Rearrangement | 1-2 |
| HER2 | Mutation | 1.6-4 |
| RET | Rearrangement | 1-2 |
| MET | Amplification | 4 |
| BRAF | Mutation | 1-3 |
| PIK3CA | Mutation | 2 |
| NTRK1 | Rearrangement | 3 |
| FGFR | Amplification | 3 |
| DDR2 | Mutation | 0.5 |

**[Table 2]**

| Human gene | Forward (5' → 3') | Reverse (5' → 3') |
|---|---|---|
| EGFR | *TGCATACAGTGCCACCCAGAG* | *GCACACTGGATACAGTTGTCTGGTC* |
| KRAS | *CAGTAGACACAAAACAGGCTCAG* | *TGTCGGATCTCCCTCACCAATG* |
| ROS1 | *GCTCAAGAACCCGACCAAAG* | *CACATCGCCATCTTCACCTTC* |
| HER2 | *GGAAGTACACGATGCGGAGACT* | *ACCTTCCTCAGCTCCGTCTCTT* |
| RET | *GTCCTCTTGCTCCACTTCAACG* | *CCTGGCAGTTTTCCACACAGAC* |
| MET | *TGCACAGTTGGTCCTGCCATGA* | *CAGCCATAGGACCGTATTTCGG* |
| BRAF | *AACGAGACCGATCCTCATCAGC* | *GGTAGCAGACAAACCTGTGGTTG* |
| PIK3CA | *GAAGCACCTGAATAGGCAAGTCG* | *GAGCATCCATGAAATCTGGTCGC* |
| NTRK1 | *GACCCCATCCCTGACACTAAC* | *AGGAAGAGGCAGGCAAAGAC* |
| FGFR1 | *GCACATCCAGTGGCTAAAGCAC* | *AGCACCTCCATCTCTTTGTCGG* |
| DDR2 | *AACGAGAGTGCCACCAATGGCT* | *ACTCACTGGCTTCAGAGCGGAA* |

Based on [Table 2], gene expression was confirmed using cDNA to determine the expression levels of genes. The mRNA expression levels of lung cancer cells (A549) were normalized to β-actin and then compared to the control group as fold changes. As a result, the culture medium and cell-free supernatant of the strain decreased the expression of all genes listed in [Table 1].

Specifically, the expression of EGFR, which causes the highest mutation in non-small cell lung cancer, was decreased, and the expression of KRAS/PI3K, a downstream regulator of EGFR was decreased.

### <Hollow fiber assay>

The sterilized 1.0mm PVDF Hollow fiber membrane (500kD) was cut into 2cm lengths, was parallelized in RPMI1640 medium containing 20% fetal bovine serum at 37°C in a 5% CO₂ incubator for 12 hours. Subsequently, a cell suspension of human non-small cell lung cancer A549 cells (1x106 cells/ml) in RPMI1640 medium containing 10% fetal bovine serum was prepared and used to fill the interior of the hollow fiber using aseptic techniques. The ends of the fibers were heat-sealed, and was cultured at 37°C in a 5% CO₂ incubator for 24 hours for stabilization.

For the experiment, a total of 5 groups (control, 500mg/kg, 1000mg/kg, 2000mg/kg, 3000mg/kg) with 3 mice per cage were used. Male athymic nude mice (BALB/c) with an average weight of 23.24±1.33g at 5 weeks of age were used. Prior to the experiment, mice were anesthetized with isoflurane inhalation anesthesia. The HF was then transplanted and sutured into the left abdominal cavity. In the control group, the same HF wasvcultured for 7 days in a 37°C 5% CO₂ incubator. For 7 days, the cell-free supernatant was orally administered at different concentrations. After 7 days, the HF was collected, washed with PBS, and cultured in pre-warmed RPMI1640 medium at 37°C for 30 minutes.

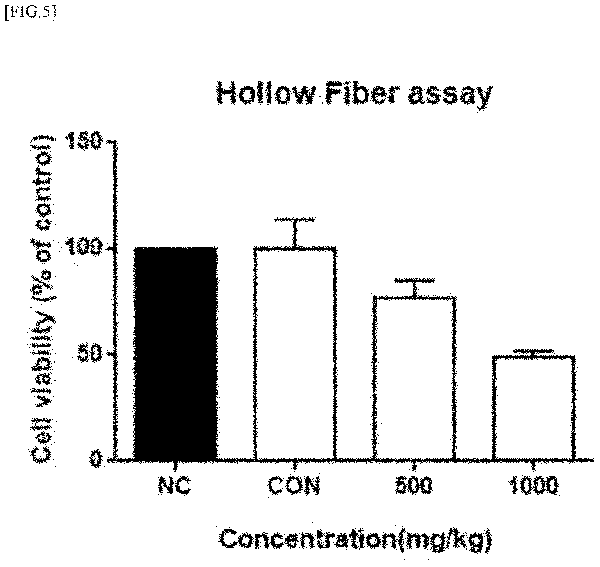

Subsequently, the HF was fixed in in a solution containing 2.5% protamine sulfate for 24 hours at 4°C after reacting with MTT (1mg/ml) at 37°C for 4 hours in a CO2 incubator, and was cut. Subsequently, the cells inside the fiber were extracted, dried, and were then reacted with DMSO (250µl) at room temperature for 4 hours with shaking. Absorbance was measured at 540nm, and the results were graphed as shown in FIG. 5.

In FIG. 5, a concentration-dependent inhibition of proliferation of non-small cell lung cancer cells was shown similar to the results of the cell viability rate experiment.

### Example 2. The apoptosis effect of Cyclo(L-pro-L-tyr), an active ingredient of the culture medium of Enterococcus faecium LCM001 strain, and its derivatives on lung cancer cells

### < Isolation of an active ingredient>

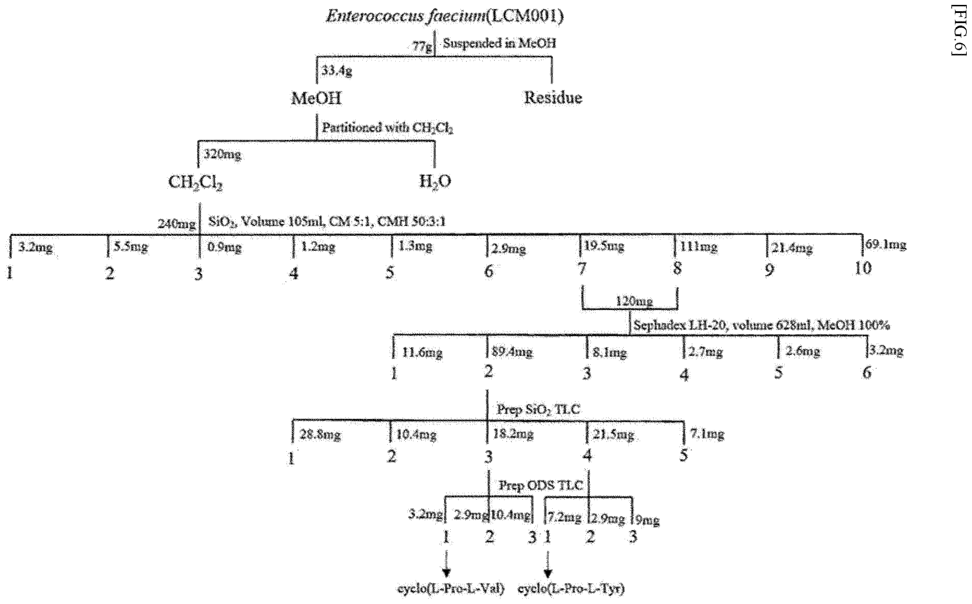

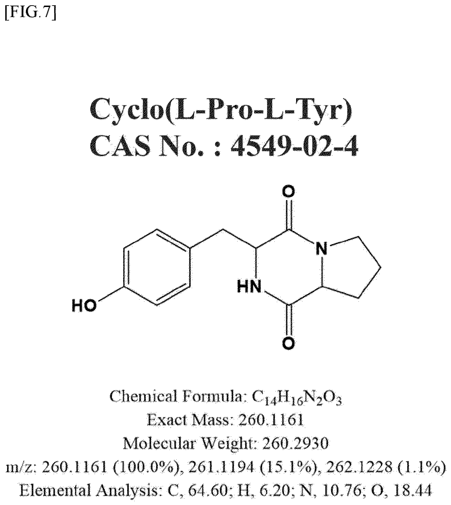

Enterococcus faecium LCM001 was cultured at 37°C and 100rpm, with a pH of 6.9-7.2, for 24 hours in 3L fermentation tank of Tryptic soy broth (TSB) that was sterilized at 121°C for 15 minutes. After the culture, the culture medium of the strain was separated by centrifugation to obtain a cell-free supernatant. The supernatant was then concentrated under reduced pressure, dissolved in 100% MeOH, and the undissolved precipitate was removed. CH₂Cl₂ was added to the MeOH fraction with same amount of the fraction, and a CH₂Cl₂ fraction was obtained. The CH₂Cl₂ fraction was passed through SiO₂ resin volume 105 ml, CM 5:1 and eluted with CMH (50:3:1) to obtain 10 fractions. Fraction 7 and 8 were further purified using a Sephadex LH-20 column to obtain 6 fractions. Fraction 2 was divided into 5 final fractions using prep SiO2 TLC, and Cyclo(L-pro-L-tyr) was finally obtained from the 1st band of fraction 4. The detailed process of the active ingredient isolation is briefly summarized in FIG. 6, and the chemical formula and physicochemical properties of Cyclo(L-pro-L-tyr), the active ingredient of Enterococcus faecium LCM001, are presented in FIG. 7.

### <Cell viability>

To confirm the efficacy of Cyclo(L-pro-L-tyr), the active ingedient of Enterococcus faecium LCM001, as well as its derivatives on apoptosis of non-small cell lung cancer A549 cells, the inventors conducted an MTT assay on Cyclo(L-pro-L-tyr), Cyclo(L-Phe-L-pro), Cyclo(L-Phe-trans-4-hydroxy-L-Pro), Cyclo(L-Pro-D-Phe), and Cyclo(Tyr-Hpro), and calculated their IC50 values.

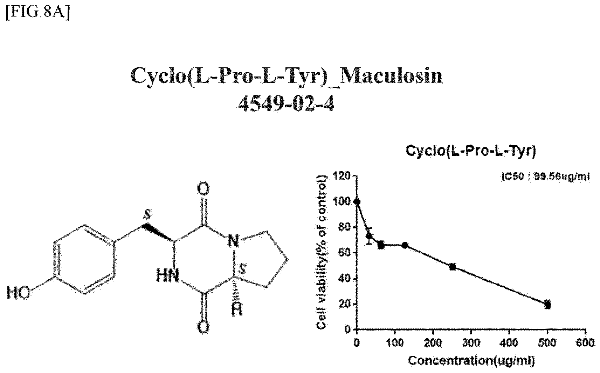

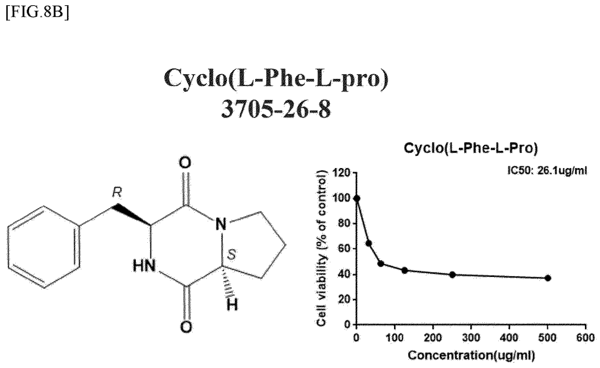

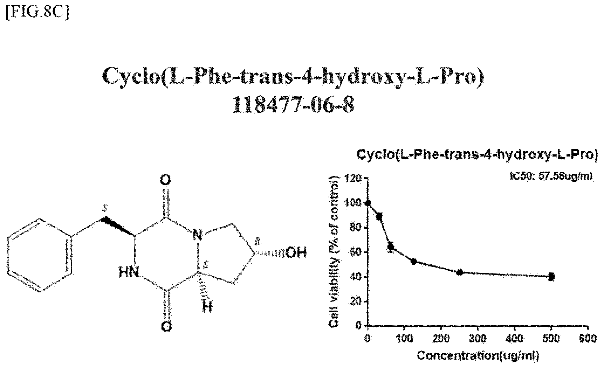

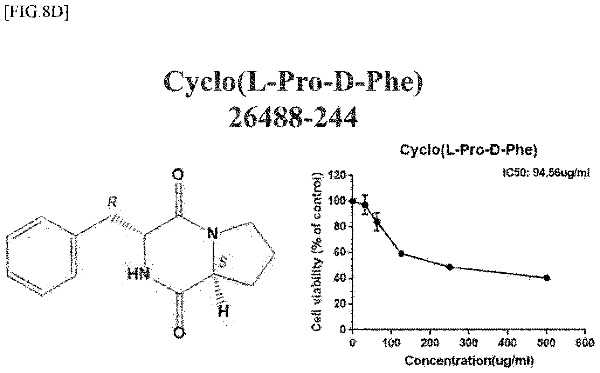

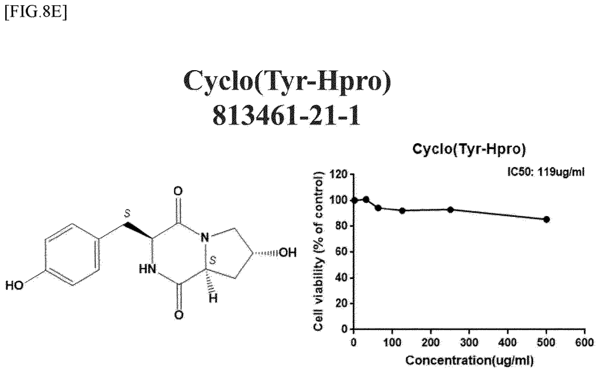

FIG. 8a-e, the IC50 value of Cyclo(L-pro-L-tyr), the active ingredient of Enterococcus faecium LCM001, was found to be 99.56 µg/ml, while the IC50 values of Cyclo(L-Phe-L-pro), Cyclo(L-Phe-trans-4-hydroxy-L-Pro), Cyclo(L-Pro-D-Phe), and Cyclo(Tyr-Hpro) were 26.1 µg/ml, 57.58 µg/ml, 94.56 µg/ml, and 119 µg/ml, respectively.

In summary, Cyclo(L-Phe-L-pro), which does not contain any -OH groups, showed the highest apoptotic efficacy, while Cyclo(Tyr-Hpro), which contains two -OH groups, showed the lowest apoptotic efficacy. Therefore, it can be concluded that ingredients with fewer -OH groups exhibit superior apoptotic activity against non-small cell lung cancer cells.

In the present specification, the present invention has been described with reference to specific embodiments, but various other embodiments are possible within the scope of the present invention. Additionally, although not described, means equivalent to those described herein may also be combined with the present invention. Therefore, the true scope of the present invention should be determined by the appended claims.

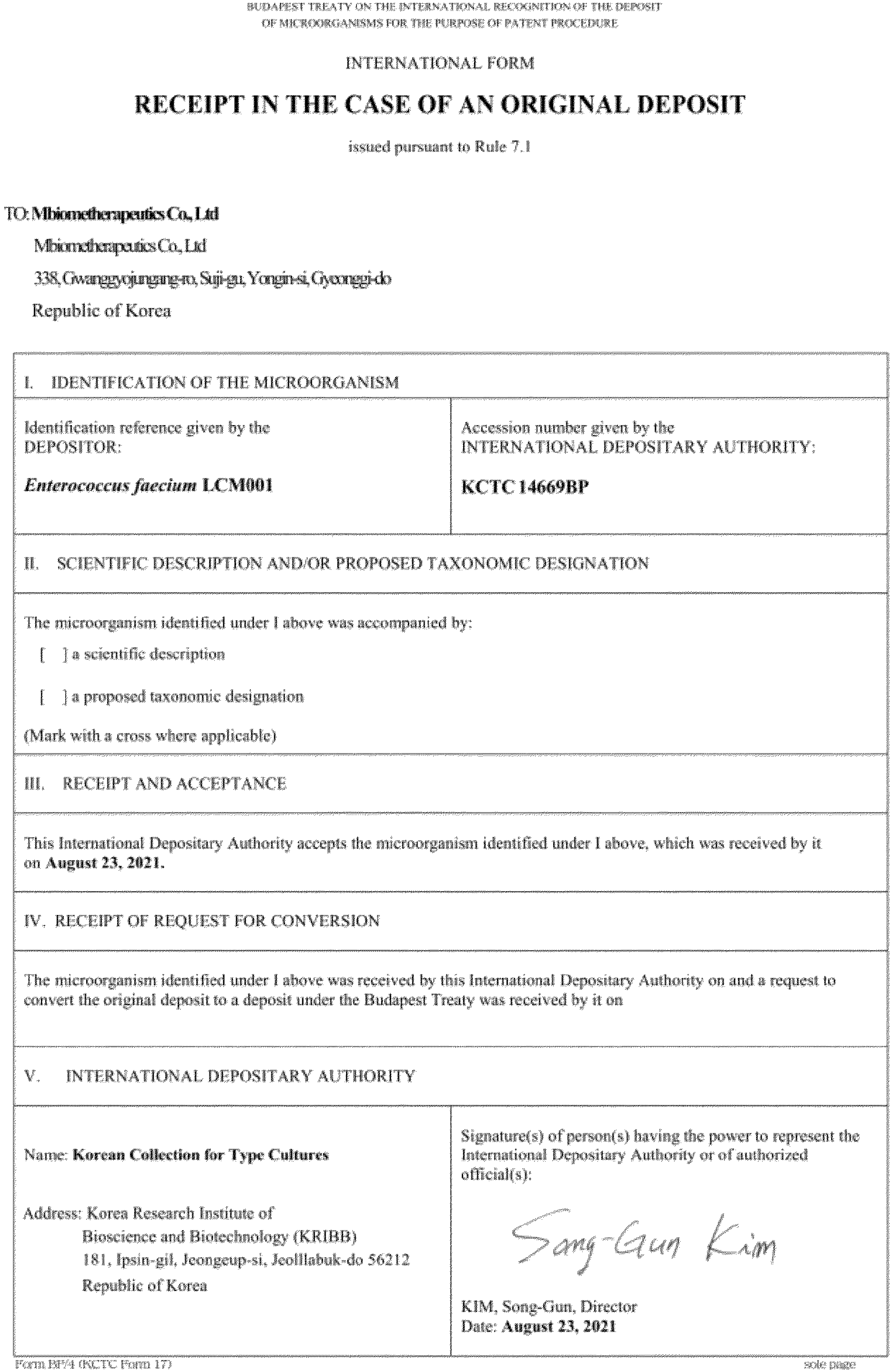
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Mbiometherapeutics Co., Ltd
Mbiometherapeutics Co., Ltd
338, Gwanggyojungang-ro, Suji-gu, Yongin-si, Gyeonggi-do
Republic of Korea

I. IDENTIFICATION OF THE MICROORGANISM
Identification reference given by the
DEPOSITOR:
Enterococcus faecium LCM001 | Accession number given by the
INTERNATIONAL DEPOSITARY AUTHORITY:
KCTC 14669BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on August 23, 2021.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):
KIM, Song-Gun, Director
Date: August 23, 2021
Form BP/4 (KCTC Form 17) sole page

## Claims

1. A novel strain of Enterococcus faecium LCM001 (Deposition number: KCTC 14669BP).

2. The strain of Claim 1, wherein the strain is **characterized by** having a 16S rRNA gene sequence [SEQ ID NO: 1].

3. The strain of Claim 1, wherein culture medium or cell-free supernatant of the strain shows apoptotic effect on cancer cells.

4. The strain of Claim 1, wherein culture medium or cell-free supernatant of the strain inhibits expression of any one of EGFR, ALK, KRAS, ROS1, HER2, RET, MET, BRAF, PIK3CA, NTRK1, FGFR, or DDR2 in cancer cells.

5. The strain of Claim 3 or 4, wherein the cancer is one or more selected from the group consisting of cervical cancer, lung cancer, pancreatic cancer, non-small cell lung cancer, liver cancer, colon cancer, colorectal cancer, bone cancer, skin cancer, brain cancer, throat cancer, skin melanoma, intraocular melanoma, uterine cancer, ovarian cancer, rectal cancer, liver cancer, brain tumor, bladder cancer, blood cancer, stomach cancer, anal cancer, breast cancer, fallopian tube cancer, endometrial cancer, vaginal cancer, vulvar cancer, Hodgkin's disease, esophageal cancer, small intestine cancer, endocrine gland cancer, thyroid cancer, parathyroid cancer, adrenal gland cancer, sarcoma, urethral cancer, penile cancer, prostate cancer, bladder cancer, kidney cancer, ureter cancer, renal cell carcinoma, renal pelvis carcinoma, central nervous system (CNS) tumors, primary CNS lymphoma, spinal cord tumors, brainstem gliomas and pituitary gland tumors.

6. The strain of Claim 3 or 4, wherein the culture medium or cell-free supernatant of the strain specifically induces apoptosis of cancer cells, not apoptosis of normal cells.

7. The strain of Claim 3 or 4, wherein the culture medium or cell-free supernatant of the strain inhibits EGFR/KRAS/PI3K pathway.

8. A composition for cancer prevention or treatment, comprising a novel strain of Enterococcus faecium LCM001 (Deposition number: KCTC 14669BP), culture medium, or cell-free supernatant of the strain as an active ingredient.

9. The strain of Claim 8, wherein the active ingredient inhibits expression of any one of EGFR, ALK, KRAS, ROS1, HER2, RET, MET, BRAF, PIK3CA, NTRK1, FGFR, or DDR2 in cancer cells.

10. a composition for cancer prevention or treatment, comprising a compound [Chemical Formula 1] as an active ingredient: 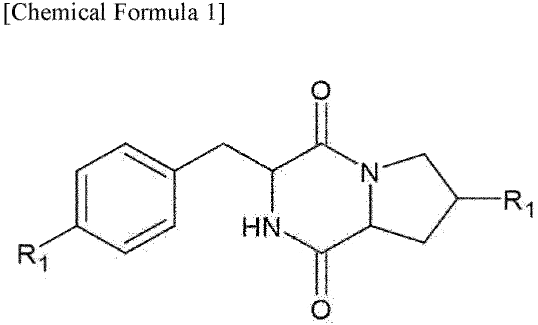 R1 is H or OH, independently.

11. The composition of Claim 10, wherein the compound [Chemical Formula 1] is isolated from culture medium of a novel strain of Enterococcus faecium LCM001 (Deposition number: KCTC 14669BP).

12. The composition of Claim 10, wherein the compound [Chemical Formula 1] inhibits expression of any one of EGFR, ALK, KRAS, ROS1, HER2, RET, MET, BRAF, PIK3CA, NTRK1, FGFR, or DDR2 in cancer cells.

13. The composition of Claim 10, wherein the compound is any one of [Chemical Formula 2] to [Chemical Formula 6]:

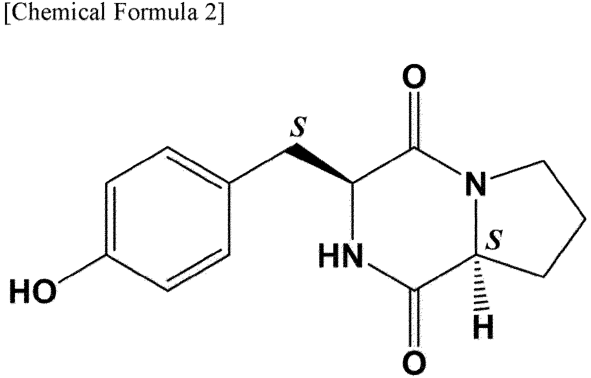

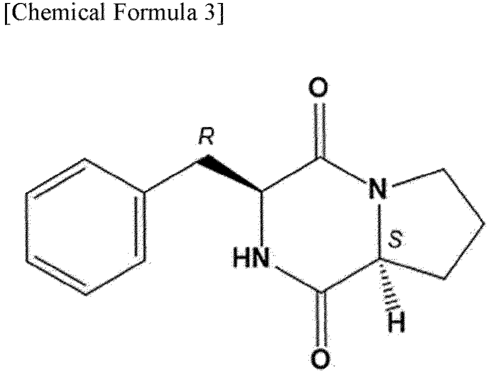

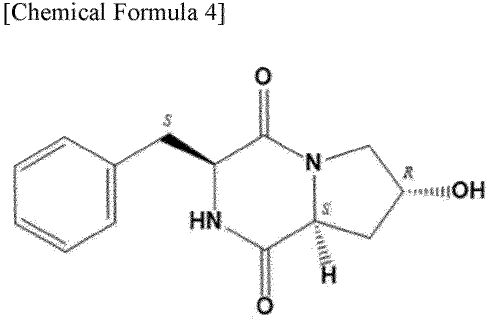

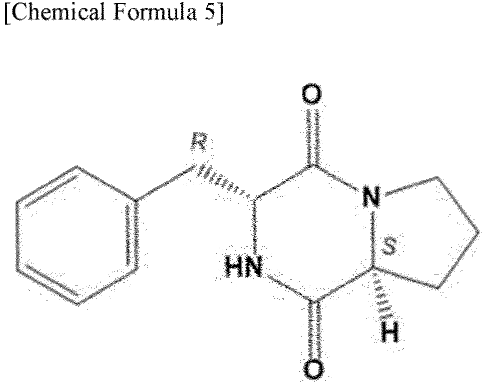

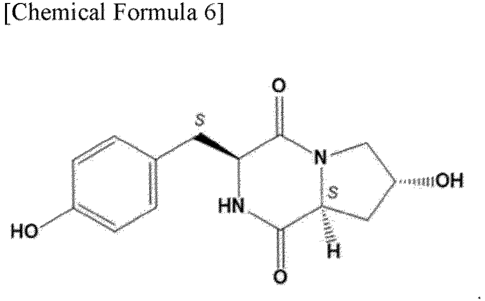

14. Health functional food for cancer prevention, comprising a novel strain of Enterococcus faecium LCM001 (Deposition number: KCTC 14669BP), culture medium, or cell-free supernatant of the strain as an active ingredient.

15. Health functional food for cancer prevention, comprising a compound [Chemical Formula 1] as an active ingredient: 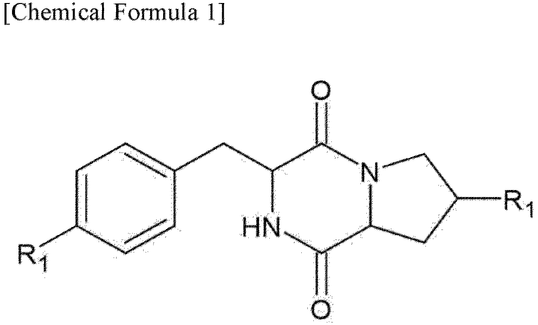 R1 is H or OH, independently.
